# EUROPEAN PATENT SPECIFICATION

(11) **EP 4 604 886 B1**
(45) Date of publication and mention of the grant of the patent: **15.07.2026**
(21) Application number: 24812975.1
(22) Date of filing: 04.11.2024
(51) Int. Cl.: A61F 5/44, A61F 5/445

(54) **DRAINABLE OSTOMY POUCH OUTLET**
AUSLASS FÜR DRAINIERBARE STOMABEUTEL
DÉCHARGE POUR POCHE DE STOMIE DRAINANTE

(30) Priority: 06.11.2023 US 202363596437 P
(43) Date of publication of application: 27.08.2025
(73) Proprietor: HOLLISTER INCORPORATED, Libertyville, Illinois 60048 (US)
(72) Inventor: O'MALLEY, Shane, Libertyville, Illinois 60048 (US); CHRISTIANSEN, Frederik B., Libertyville, Illinois 60048 (US); WOHLGEMUTH, Jan, Libertyville, Illinois 60048 (US); NIELSEN, Kenneth, Libertyville, Illinois 60048 (US)
(74) Representative: FRKelly
(86) International application number: PCT/US2024/054398
(87) International publication number: WO 2025/101458

(56) References cited:
- EP-A2- 2 243 707
- US-A- 5 037 138
- US-A1- 2023 320 890
- US-B2- 11 612 510
- US-B2- 9 011 395

## Description

### BACKGROUND

This disclosure is related to a drainable ostomy pouch outlet.

Ostomy pouches for collecting body waste are used by patients who have had surgery such as a colostomy, ileostomy, or urostomy. Ostomy pouches typically include flat, opposing side walls secured together along their edges to define a collection cavity. One of the side walls is provided with an opening to receive a stoma and means to secure the pouch to the user, such as an adhesive barrier, so that body waste discharged through the stoma is received within the cavity.

The ostomy pouch may be a closed-end pouch for single use, in which case the entire pouch is discarded after it has been substantially filled with stomal discharge. Alternatively, the ostomy pouch can be a drainable pouch with a discharge opening at its lower end, which may be closed during the collection of body waste material but may be opened for draining body waste material from the pouch after a period of use. The discharge opening of drainable pouches is typically defined at the end of a narrowed outlet portion, which is provided with closure means for maintaining the discharge opening in a sealed condition until waste material is to be drained from the pouch. However, the discharge opening may be difficult to open and close properly to ensure that a good seal is provided upon closing.

US11612510B2 discloses a drainable ostomy pouch outlet including a single-piece closure member including at least one transversely-extending fold-line. The at least one transversely-extending fold-lines are configured to facilitate folding up and closing of the outlet. The single-piece closure member may also include at least one axially-extending or slanted fold-lines intersecting the at least one transversely-extending fold-line, configured to facilitate opening of the outlet.

Accordingly, there is a need for an improved drainable pouch outlet.

### BRIEF SUMMARY

A drainable pouch is provided according to various embodiments.

In one aspect, the drainable pouch can include a barrier side wall, a distal wall, an outlet defining a discharge opening, and at least one closure member located on and attached to a surface of the outlet and having a kink line extending across the closure member and protruding in a direction away from the surface of the outlet. The barrier side wall and the distal wall can be joined along their peripheral edges to define a cavity and to define the outlet, the outlet being foldable upward toward the cavity. The at least one closure member may be configured to bend in the direction away from the surface of the outlet when opening the outlet.

In an embodiment, the drainable pouch can further include a securing member located on a barrier side of the outlet and a cover flap located on a distal side of the outlet for receiving and securing the securing member when the outlet is folded up.

In an embodiment, the at least one closure member can bend and open the outlet when a force beyond a threshold force is applied to outer edges of the at least one closure member.

In an embodiment, the at least one closure member can include first and second closure members. The first closure member can be located on the barrier side of the outlet, and the second closure member can be located on the distal side of the outlet. In such an embodiment, the first closure member can be located near a discharge opening of the outlet. The second closure member can be positioned to align with the first closure member and to overlie a portion of the outlet at which the first closure member is located, when the outlet is folded up.

In an embodiment, the at least one closure member may comprise a first closure member including a first kink line extending across the first closure member and protruding in a direction away from a barrier side surface of the outlet and a second closure member including a second kink line extending across the second closure member and protruding in a direction away from a distal surface of the outlet. In this embodiment, the first closure member may be attached to the barrier side wall of the outlet and the second closure member may be attached to the distal wall of the outlet and arranged vertically spaced apart from the first closure member, such that an accordion-like folds may be formed in the outlet. The first and second closure members may be configured and arranged such that the first closure member nests over the second closure member after a first folding up of the outlet. The first and second closure members may be configured to provide a tactile feedback when first closure member correctly nests over the second closure member to facilitate leak-free closure of the outlet.

In an embodiment, the first and second kink lines may be formed across the corresponding closure members in a direction perpendicular to the direction of the outlet extending from the cavity. The first and second closure members may be configured to bend away from the outlet in opposite directions.

The kink line may be formed by a fold line extending across the corresponding closure member and protruding away from the outlet or a crease extending across the corresponding closure member.

In some embodiments, each of the at least one closure member may include a cross-kink line arranged approximately in a midpoint of the closure member and configured to facilitate the closure member to bend at the midpoint in a direction away from the outlet when an inward force is applied to outer edges of the closure member to open the outlet. The cross-kink line may extend vertically in the direction of the outlet extending from the cavity. The cross-kink line may be formed by a fold line, score line, crease line, or an embossed line.

In an embodiment, the drainable pouch can further include a tab. The tab may be attached to a surface of the outlet and configured to facilitate opening the outlet to drain the cavity.

In the any of foregoing embodiments, the at least one closure member may be formed from a polymeric material.

The foregoing general description and the following detailed description are examples only and are not restrictive of the present disclosure.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a barrier side view of an outlet portion of an ostomy pouch in an unfolded position, according to an embodiment.
FIG. 2 is a distal side view of the outlet portion of the ostomy pouch of FIG. 1 in an unfolded position.
FIG. 3A is a schematic cross-sectional view of the outlet portion of the ostomy pouch of FIG. 1 in an open position.
FIG. 3B is a schematic cross-sectional view of the outlet portion of the ostomy pouch of FIG. 1 folded up one time.
FIG. 3C is a schematic cross-sectional view of the outlet portion of the ostomy pouch of FIG. 1 folded up two times.
FIG. 3D is a schematic cross-sectional view of the outlet portion of the ostomy pouch of FIG. 1 folded up three times.
FIG. 3E is a schematic cross-sectional view of the outlet portion of the ostomy pouch of FIG. 1 folded up three times and secured in place with a pair of connected covers.
FIG. 4 is a distal side view of an outlet portion of an embodiment of an ostomy pouch in an unfolded position.
FIG. 5 is a bottom view of the outlet portion of the ostomy pouch of FIG. 4.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular. The words "first," "second," "third," and the like may be used in the present disclosure to describe various information, such information should not be limited to these words. These words are only used to distinguish one category of information from another. The directional words "top," "bottom," up," "down," front," "back," and the like are used for purposes of illustration and as such, are not limiting. Depending on the context, the word "if" as used herein may be interpreted as "when" or "upon" or "in response to determining."

The present disclosure provides a drainable ostomy pouch that includes a drainage outlet that is easy to open and clean. The drainage outlet may include one or more closure members with a kink line that allows the drainage outlet to be correctly opened at a midpoint for cleaning and securely closed to ensure a good seal of the pouch outlet.

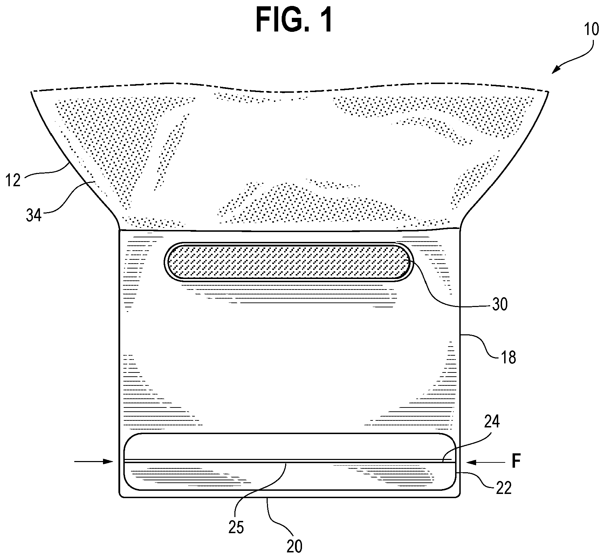

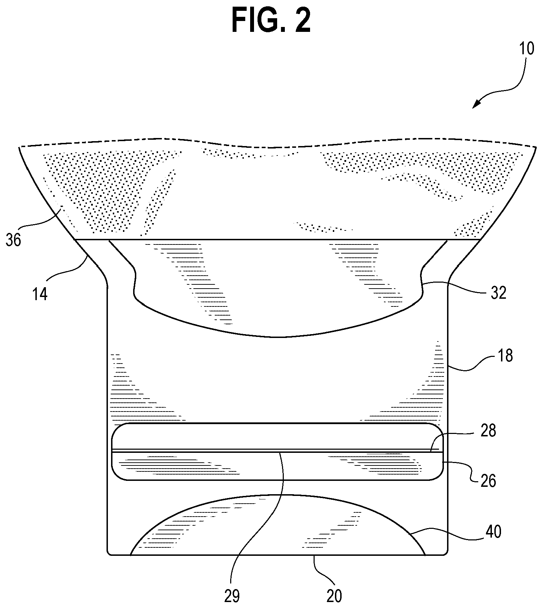

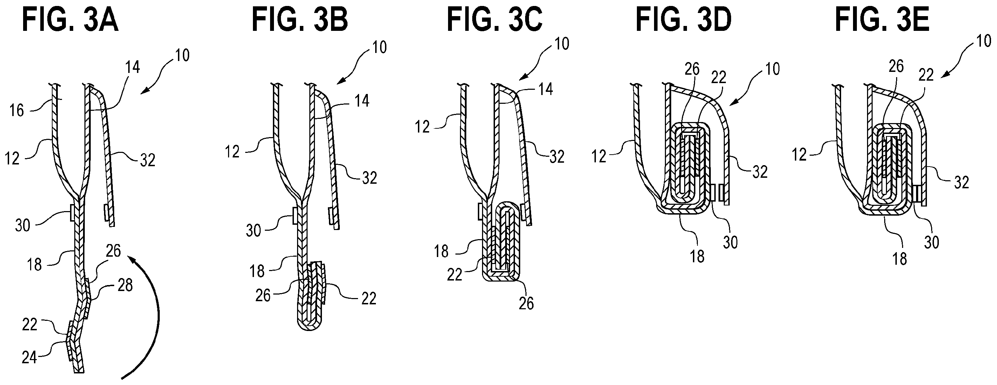

Referring now to the figures, FIGS. 1-5 a drainable pouch 10, 110 according to multiple embodiments. The drainable pouch 10, 110 can generally include a barrier or body side wall 12, a distal side wall 14, 114, a cavity 16, an outlet 18, 118, a discharge opening 20, 120, a first closure member 22 having a first kink line 24, a second closure member 26, 126 having a second kink line 28, 128, a securing member 30, a cover flap 32, 132, a barrier side cover layer 34, and a distal side cover layer 36, 136. In the embodiment shown in FIG. 2, the distal side wall 14, 114 of the outlet 18, 118 may include a distal side cutout 40, 140. The barrier side wall 12 and the distal side wall 14 can be joined along their peripheral edges to define the cavity 16 therebetween for collecting stomal discharge or effluent. The outlet 18 can terminate in a discharge opening 20 for draining the contents collected in the cavity after a period of use. The discharge opening 20 of the drainable pouch 10, 110 may be closed during use by folding outlet 18 upwardly (as shown in FIGS. 3A-3D) and securing it in the upwardly folded position as shown in FIG. 3E. The drainable pouch 10, 110, for example, may be a drainable ostomy pouch.

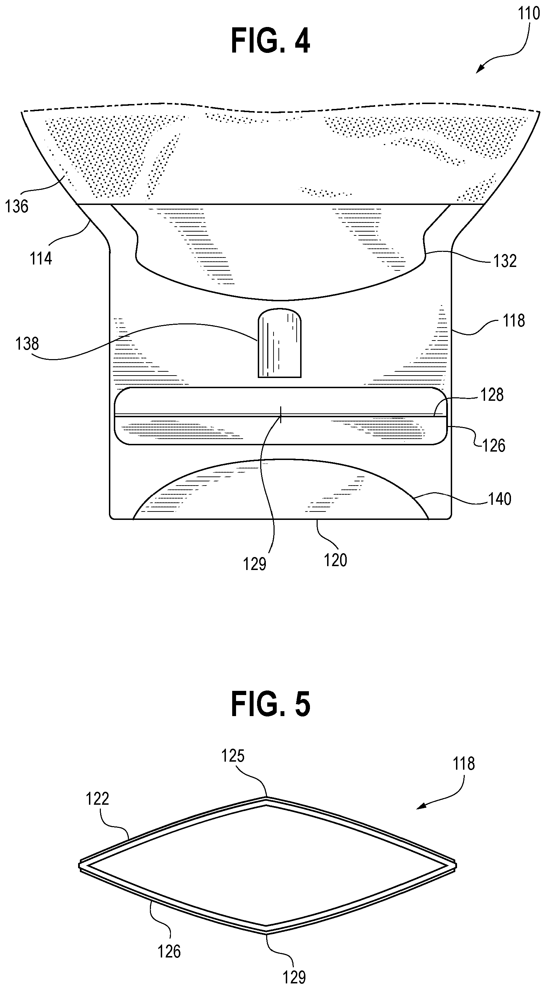

The closure members 22, 26 may be formed from a relatively flexible material (e.g. a polymeric material) and configured to allow the closure members 22, 26 to be bent at about their respective midpoints 25, 29 by applying an inward force F at their outer edges (FIGS. 1 and 5). As such, the closure members 22, 26 can function as living hinges, integral hinges, or other types of flexible hinges. The closure members 22, 26 may be arranged and configured to be bent in opposite directions at the midpoints 25, 29 to open the outlet 18. The kink lines 24, 28 may be configured to extend away from the outlet 18 and prevent the closure members 22, 26 from bending into the outlet 18.

In an embodiment, the closure members 22, 26 can be used to open the outlet 18 by applying pressure F on the outer edges of the closure members 22, 26, so that the closure members 22, 26 may bend or flex outwardly approximately at the midpoints 25, 29. In such an embodiment, as a threshold pressure is applied, the closure members 22, 26 may bend away from each other at their midpoints to open the outlet 18 and the discharge opening 20. The amount of force needed to bend the closure members 22, 26 and open the outlet 18, for example, the threshold pressure, can be such that the closure members 22, 26 are not inadvertently bent, for example, during an undesirable situation. The closure members 22, 26 can stay closed or in a resting position when pressure is not applied to the closure members 22, 26. The closed or resting position can prevent or reduce the risk of effluent leaking from the outlet 18 when the outlet 18 is being unfolded. When pressure is released from the sides of the closure members 22, 26, the closure members 22, 26 can return to their resting position and the outlet 18 may be closed while providing visual and tactile feedback to the user.

FIG. 1 illustrates a barrier side view of the outlet portion of the ostomy pouch 10. The first closure member 22 can be located near the end of the drainable pouch 10 on the outlet 18 and can extend across the outlet 18 in a direction perpendicular to the direction the outlet 18 extends from the drainable pouch 10. The first closure member 22 can further include the kink line 24 located along a mid-section of the first closure member 22, across the first closure member 22, and extending away from the outlet 18. The first closure member 22 can couple to the second closure member 26 to close the outlet 18 (FIGS. 3B-D). The securing member 30 can be located on the outlet 18 opposite (i.e., above) the discharge opening 20 and can extend across the outlet 18 in a direction perpendicular to the direction the outlet 18 extends from the drainable pouch 10. The securing member 30 can couple to the cover flap 32 to secure the outlet closed (FIG. 3E). The barrier cover layer 34 can be located over the barrier side wall 12 and end before the outlet 18.

FIG. 2 illustrates a distal side view of the outlet portion of the ostomy pouch 10. The second closure member 26 can be located near a mid-section of the outlet 18 to allow the first closure member 22 to fold over the second closure member 26 (FIG. 3B). The second closure member 26 can extend across the outlet 18 in a direction perpendicular to the direction the outlet 18 extends from the drainable pouch 10. The second closure member 26 can further include the kink line 28 located along a mid-section of the second closure member 26, across the second closure member 26, and extending away from the outlet 18. The cover flap 32 can be located on the ostomy pouch 10, near the outlet 18, and can extend over the outlet 18 to receive the folded-up outlet 18 and secure the outlet 18 with the secure member 30 (FIG. 3E). The discharge opening 20 can include a cutout 40, such as the illustrated arch shaped cutout, to facilitate opening the discharge opening and draining the ostomy pouch 10. The distal cover layer 36 can be located over the distal side wall 14 and end before the outlet 18. In an embodiment, the distal cover layer 36 can be located over the distal side wall 14 and can extend towards the outlet 18 up to the location of the cover 32.

In an embodiment, each of the kink lines 24, 28 may be defined by a fold line extending across the corresponding closure member 22, 26 and protruding away from the outlet 18. The closure members 22, 26 may be arranged on and attached to the opposing walls 12, 14 of the outlet 18 and configured to bend away from the outlet 18 along the kink lines 24, 28 in opposite directions to facilitate opening of the outlet 18. In the embodiments of FIGS. 1-3E, the closure members 22, 26 may be arranged vertically spaced apart on the opposite surfaces of the outlet 18, such that an accordion-like cross-sectional view can be observed in FIG. 3A. The closure members 22, 26 may be arranged such that the first closure member 22 can be stacked on top of and nested with the second closure member 26 as shown in FIG. 3B. Such a configuration of the closure members 22, 26 can facilitate correct folding up of the outlet 18 by providing a tactile feedback when the first closure member 22 correctly nests over the first closure member 26 to provide a leak-free closure of the outlet 18. In some embodiments, the kink lines 24, 28 may be defined by score lines, crease lines, embossed lines and the like that are configured to facilitate the closure members 22, 26 to bend away from the outlet 18 along the kink lines 24, 28.

FIGS. 3A-3E illustrate schematic cross-sectional views of the ostomy pouch 10. FIG. 3A illustrates the outlet 18 in an unfolded position. The ostomy pouch 10 may be closed by folding up the outlet 18 toward the distal side wall 14 so that the closure member 22 aligns with closure member 26 and overlies that portion of the outlet 18 on which closure member 26 is located, as shown in FIG. 3B. FIGS. 3B and 3C illustrate the outlet 18 in a partially rolled-up state, and FIG. 3D illustrates the outlet 18 in a fully rolled-up state. FIG. 3E shows the outlet 18 in a fully rolled up state and in a secured position. The securing member 30 can be secured under the cover flap 32 via fasteners, such ashook-and-loop fasteners. In an embodiment, the securing member 30 and the cover flap 32may be provided with hook-and-loop fasteners for securing the outlet 18 in the rolled up and closed position.

FIG. 4 illustrates a distal side view of an outlet portion of an embodiment of an ostomy pouch 110. The ostomy pouch 110 can include a tab 138 located on the outlet 118. The tab 138 can be used to facilitate opening to outlet 118 to empty the pouch 110 and/or to clean the outlet 118. For example, the tab 138 can be used to open the outlet 118 to drain effluent from the discharge opening 120. In an embodiment, the tab 138 can be located above or below the second closure member 126 (between the second closure member 126 and the outlet 118). In an embodiment, the tab 138 can include a free end oriented towards the ostomy pouch 110 (i.e., upwardly). Alternatively, the tab 138 can include a free end oriented towards a side of the outlet 118 (at an orientation perpendicular to the outlet 118). In use, a user may grab and pull the tab 138 away from the surface of the outlet 118 to facilitate opening of the outlet 118.FIG. 5 illustrates a bottom view of the outlet 118 in an open position. The closure members 122, 126 of the outlet 118 may be configured similar to the closure members 22, 26 of the outlet 18 described above, except in the embodiment of FIGS. 4 and 5, the closure members 122, 126 may be provided with cross-kink lines 125, 129 configured to facilitate the closure members 122, 126 to bend at the cross-kink lines 125, 129 when an inward force is applied to the outer edges of the closure members 122, 126 to open the outlet 118 as shown in FIG. 5. In an embodiment, each of the cross-kink lines 125, 129 may be formed by a fold line that crosses the corresponding kink-lines 128. In other embodiments, the cross-kink lines 125, 129 may be formed by score lines, crease line, embossed lines and the like.

From the foregoing it will be observed that numerous modifications and variations can be effectuated without departing from the scope of the novel concepts of the present disclosure. It is to be understood that no limitation with respect to the specific embodiments illustrated is intended or should be inferred. The disclosure is intended to cover by the appended claims all such modifications as fall within the scope of the claims.

## Claims

1. A drainable ostomy pouch (10, 110), comprising: a barrier side wall (12); a distal wall (14, 114); an outlet (18, 118) defining a discharge opening (20, 120), wherein the barrier side wall and the distal wall are joined along their peripheral edges to define a cavity (16) and to define the outlet, the outlet being foldable upward toward the cavity; and at least one closure member (22) located on and attached to a surface of the outlet and having a kink line (24) extending across the closure member (22), wherein the at least one closure member (22) is configured to bend in the direction away from the surface of the outlet when opening the outlet, **characterised in that** the kink line (24) protrudes in a direction away from the surface of the outlet (18, 118).

2. The drainable ostomy pouch (10) of claim 1, further comprising: a securing member (30) located on the barrier side wall (12) of the outlet and a cover flap (32, 132) located on the distal side wall of the outlet for receiving and securing the securing member when the outlet is folded up.

3. The drainable ostomy pouch (10) of claim 1 or 2, wherein the at least one closure member (22) is configured to bend and open the outlet (18, 118) when a force beyond a threshold force is applied to outer edges of the at least one closure member (22).

4. The drainable ostomy pouch (10) of any one of claims 1-3, wherein the at least one closure member (22) comprises first (22) and second (26, 126) closure members, the first and second closure members including first and second kink lines (24, 28, 128), respectively, and wherein the first closure member (22) is located on the barrier side wall of the outlet, and wherein the second closure member (26, 126) is located on the distal side wall of the outlet.

5. The drainable ostomy pouch (10) of claim 4, wherein the first closure member (22) is located near the discharge opening, and wherein the second closure member (26, 126) is positioned to align with the first closure member and to overlie a portion of the outlet at which the first closure member is located, when the outlet is folded up.

6. The drainable ostomy pouch (10) of any one of claims 1-3, wherein the at least one closure member (22) comprises a first closure member (22) including a first kink line (24) extending across the first closure member and protruding in a direction away from a barrier side surface of the outlet (18, 118) and a second closure member (26, 126) including a second kink line (28, 128) extending across the second closure member and protruding in a direction away from a distal surface of the outlet, wherein the first closure member (22) is attached to the barrier side wall of the outlet and the second closure member (26, 126) is attached to the distal wall of the outlet and arranged vertically spaced apart from the first closure member such that an accordion-like folds are formed in the outlet.

7. The drainable ostomy pouch (10) of claim 6, wherein the first and second closure members (22, 26, 126) are configured and arranged such that the first closure member (22) nests over the second closure member (26, 126) after a first folding up of the outlet.

8. The drainable pouch (10) of claim 7, wherein the first and second closure members (22, 26, 126) are configured to provide a tactile feedback when first closure member (22) correctly nests over the second closure member (26, 126) to facilitate leak-free closure of the outlet.

9. The drainable ostomy pouch (10) of any one of claims 7-8, wherein the first and second kink lines (24, 28, 128) are formed across the corresponding closure members (22, 26, 126) in a direction perpendicular to the direction of the outlet extending from the cavity.

10. The drainable ostomy pouch (10) of any one of claims 4-9, wherein the first and second closure members (22, 26, 126) are configured to bend away from the outlet in opposite directions.

11. The drainable ostomy pouch (10) of any one of claims 1-10, wherein the kink line (24, 28, 128) is formed by a fold line extending across the corresponding closure member (22, 26, 126) and protruding away from the outlet (18, 118), and/or wherein the kink line (24, 28, 128) is defined by a crease extending across the corresponding closure member.

12. The drainable ostomy pouch (10) of any one of claims 1-11, wherein each of the at least one closure member (22, 26, 126) includes a cross-kink line (125, 129) arranged approximately in a midpoint of the closure member (22, 26, 126) and configured to facilitate the closure member to bend at the midpoint in a direction away from the outlet when an inward force is applied to outer edges of the closure member to open the outlet (18, 118).

13. The drainable ostomy pouch (10) of claim 12, wherein the cross-kink line (125, 129) extends vertically in the direction of the outlet extending from the cavity (16), and/or wherein the cross-kink line is formed by a fold line, score line, crease line, or an embossed line.

14. The drainable ostomy pouch (10) of any one of claims 1-13 wherein the at least one closure member (22, 26, 126) is formed from a polymeric material.

15. The drainable ostomy pouch (10) of any one of claims 1-14, further comprising: a tab (138) attached to a surface of the outlet (18, 118) and configured to facilitate opening the outlet to drain the cavity.

## Patentansprüche

1. Drainierbarer Stomabeutel (10, 110), umfassend: eine barriereseitige Wand (12); eine distale Wand (14, 114); einen Auslass (18, 118), der eine Abgabeöffnung (20, 120) definiert, wobei die barriereseitige Wand und die distale Wand entlang ihrer Umfangskanten verbunden sind, um einen Hohlraum (16) zu definieren und um den Auslass zu definieren, wobei der Auslass nach oben in Richtung des Hohlraums faltbar ist; und mindestens ein Verschlusselement (22), das auf einer Oberfläche des Auslasses angeordnet und an dieser befestigt ist und eine Knicklinie (24) aufweist, die sich über das Verschlusselement (22) erstreckt, wobei das mindestens eine Verschlusselement (22) so konfiguriert ist, dass es sich beim Öffnen des Auslasses in der Richtung weg von der Oberfläche des Auslasses biegt, **dadurch gekennzeichnet, dass** die Knicklinie (24) in einer Richtung weg von der Oberfläche des Auslasses (18, 118) vorsteht.

2. Drainierbarer Stomabeutel (10) nach Anspruch 1, ferner umfassend: ein Befestigungselement (30), das an der barriereseitigen Wand (12) des Auslasses angeordnet ist, und eine Abdeckklappe (32, 132), die an der distalen Seitenwand des Auslasses angeordnet ist, um das Befestigungselement aufzunehmen und zu sichern, wenn der Auslass hochgefaltet ist.

3. Drainierbarer Stomabeutel (10) nach Anspruch 1 oder 2, wobei das mindestens eine Verschlusselement (22) so konfiguriert ist, dass es sich biegt und den Auslass (18, 118) öffnet, wenn eine Kraft über eine Schwellenkraft hinaus auf Außenkanten des mindestens einen Verschlusselements (22) ausgeübt wird.

4. Drainierbarer Stomabeutel (10) nach einem der Ansprüche 1-3, wobei das mindestens eine Verschlusselement (22) erste (22) und zweite (26, 126) Verschlusselemente umfasst, wobei das erste und das zweite Verschlusselement erste bzw. zweite Knicklinien (24, 28, 128) beinhalten, und wobei das erste Verschlusselement (22) an der barriereseitigen Wand des Auslasses angeordnet ist, und wobei das zweite Verschlusselement (26, 126) an der distalen Seitenwand des Auslasses angeordnet ist.

5. Drainierbarer Stomabeutel (10) nach Anspruch 4, wobei das erste Verschlusselement (22) nahe der Abgabeöffnung angeordnet ist, und wobei das zweite Verschlusselement (26, 126) so positioniert ist, dass es mit dem ersten Verschlusselement fluchtet und einen Abschnitt des Auslasses überlagert, an dem sich das erste Verschlusselement befindet, wenn der Auslass hochgefaltet ist.

6. Drainierbarer Stomabeutel (10) nach einem der Ansprüche 1-3, wobei das mindestens eine Verschlusselement (22) ein erstes Verschlusselement (22) umfasst, das eine erste Knicklinie (24) beinhaltet, die sich über das erste Verschlusselement erstreckt und in einer Richtung weg von einer barriereseitigen Oberfläche des Auslasses (18, 118) vorsteht, und ein zweites Verschlusselement (26, 126) umfasst, das eine zweite Knicklinie (28, 128) beinhaltet, die sich über das zweite Verschlusselement erstreckt und in einer Richtung weg von einer distalen Oberfläche des Auslasses vorsteht, wobei das erste Verschlusselement (22) an der barriereseitigen Wand des Auslasses befestigt ist und das zweite Verschlusselement (26, 126) an der distalen Wand des Auslasses befestigt und vertikal beabstandet von dem ersten Verschlusselement angeordnet ist, so dass ein akkordeonartige Falten in dem Auslass gebildet werden.

7. Drainierbarer Stomabeutel (10) nach Anspruch 6, wobei das erste und das zweite Verschlusselement (22, 26, 126) so konfiguriert und angeordnet sind, dass das erste Verschlusselement (22) nach einem ersten Hochfalten des Auslasses über dem zweiten Verschlusselement (26, 126) nistet.

8. Drainierbarer Beutel (10) nach Anspruch 7, wobei das erste und das zweite Verschlusselement (22, 26, 126) so konfiguriert sind, dass sie eine taktile Rückmeldung bereitstellen, wenn erstes Verschlusselement (22) korrekt über dem zweiten Verschlusselement (26, 126) nistet, um einen leckagefreien Verschluss des Auslasses zu erleichtern.

9. Drainierbarer Stomabeutel (10) nach einem der Ansprüche 7-8, wobei die erste und die zweite Knicklinie (24, 28, 128) über die entsprechenden Verschlusselemente (22, 26, 126) in einer Richtung senkrecht zu der Richtung des Auslasses, der sich aus dem Hohlraum erstreckt, gebildet sind.

10. Drainierbarer Stomabeutel (10) nach einem der Ansprüche 4-9, wobei das erste und das zweite Verschlusselement (22, 26, 126) so konfiguriert sind, dass sie sich in entgegengesetzten Richtungen weg von dem Auslass biegen.

11. Drainierbarer Stomabeutel (10) nach einem der Ansprüche 1-10, wobei die Knicklinie (24, 28, 128) durch eine Faltlinie gebildet wird, die sich über das entsprechende Verschlusselement (22, 26, 126) erstreckt und von dem Auslass (18, 118) weg vorsteht, und/oder wobei die Knicklinie (24, 28, 128) durch eine Falte definiert ist, die sich über das entsprechende Verschlusselement erstreckt.

12. Drainierbarer Stomabeutel (10) nach einem der Ansprüche 1-11, wobei jedes des mindestens einen Verschlusselements (22, 26, 126) eine Querknicklinie (125, 129) beinhaltet, die ungefähr in einem Mittelpunkt des Verschlusselements (22, 26, 126) angeordnet und so konfiguriert ist, dass sie das Verschlusselement erleichtert, sich am Mittelpunkt in einer Richtung weg von dem Auslass zu biegen, wenn eine nach innen gerichtete Kraft auf Außenkanten des Verschlusselements ausgeübt wird, um den Auslass (18, 118) zu öffnen.

13. Drainierbarer Stomabeutel (10) nach Anspruch 12, wobei sich die Querknicklinie (125, 129) vertikal in der Richtung des Auslasses erstreckt, der sich aus dem Hohlraum (16) erstreckt, und/oder wobei die Querknicklinie durch eine Faltlinie, Rilllinie, Faltlinie oder eine geprägte Linie gebildet wird.

14. Drainierbarer Stomabeutel (10) nach einem der Ansprüche 1-13 wobei das mindestens eine Verschlusselement (22, 26, 126) aus einem Polymermaterial gebildet ist.

15. Drainierbarer Stomabeutel (10) nach einem der Ansprüche 1-14, ferner umfassend: eine Lasche (138), die an einer Oberfläche des Auslasses (18, 118) befestigt und so konfiguriert ist, dass sie das Öffnen des Auslasses erleichtert, um den Hohlraum zu drainieren.

## Revendications

1. Poche de stomie drainante (10, 110), comprenant : une paroi latérale de barrière (12) ; une paroi distale (14, 114) ; une décharge (18, 118) définissant une ouverture de décharge (20, 120), dans laquelle la paroi latérale de barrière et la paroi distale sont jointes le long de leurs bords périphériques pour définir une cavité (16) et pour définir la décharge, la décharge étant pliable vers le haut en direction de la cavité ; et au moins un élément de fermeture (22) situé sur et fixé à une surface de la décharge et présentant une ligne de pliure (24) s'étendant sur l'élément de fermeture (22), dans laquelle l'au moins un élément de fermeture (22) est configuré pour se plier dans la direction opposée à la surface de la décharge lors de l'ouverture de la décharge, **caractérisée en ce que** la ligne de pliure (24) fait saillie dans une direction opposée à la surface de la décharge (18, 118).

2. Poche de stomie drainante (10) de la revendication 1, comprenant en outre : un élément de fixation (30) situé sur la paroi latérale de barrière (12) de la décharge et un rabat de couverture (32, 132) situé sur la paroi latérale distale de la décharge pour recevoir et fixer l'élément de fixation lorsque la décharge est repliée.

3. Poche de stomie drainante (10) de la revendication 1 ou 2, dans laquelle l'au moins un élément de fermeture (22) est configuré pour se plier et ouvrir la décharge (18, 118) lorsqu'une force supérieure à une force seuil est appliquée aux bords extérieurs de l'au moins un élément de fermeture (22).

4. Poche de stomie drainante (10) de l'une quelconque des revendications 1 à 3, dans laquelle l'au moins un élément de fermeture (22) comprend des premier (22) et second (26, 126) éléments de fermeture, les premier et le second éléments de fermeture comportant respectivement des première et une seconde lignes de pliure (24, 28, 128), et dans laquelle le premier élément de fermeture (22) est situé sur la paroi latérale de barrière de la décharge, et dans laquelle le second élément de fermeture (26, 126) est situé sur la paroi latérale distale de la décharge.

5. Poche de stomie drainante (10) de la revendication 4, dans laquelle le premier élément de fermeture (22) est situé près de l'ouverture de décharge, et dans laquelle le second élément de fermeture (26, 126) est positionné pour s'aligner avec le premier élément de fermeture et pour recouvrir une partie de la décharge où se trouve le premier élément de fermeture, lorsque la décharge est repliée.

6. Poche de stomie drainante (10) de l'une quelconque des revendications 1 à 3, dans laquelle l'au moins un élément de fermeture (22) comprend un premier élément de fermeture (22) comportant une première ligne de pliure (24) s'étendant à travers le premier élément de fermeture et faisant saillie dans une direction opposée à une surface latérale de barrière de la décharge (18, 118) et un second élément de fermeture (26, 126) comportant une seconde ligne de pliure (28, 128) s'étendant à travers le second élément de fermeture et faisant saillie dans une direction opposée à une surface distale de la décharge, dans laquelle le premier élément de fermeture (22) est fixé à la paroi latérale de barrière de la décharge et le second élément de fermeture (26, 126) est fixé à la paroi distale de la décharge et disposé verticalement, espacé du premier élément de fermeture, de manière à former des plis en accordéon dans la décharge.

7. Poche de stomie drainante (10) de la revendication 6, dans laquelle les premier et second éléments de fermeture (22, 26, 126) sont configurés et disposés de telle sorte que le premier élément de fermeture (22) s'emboîte sur le second élément de fermeture (26, 126) après un premier pliage de la décharge.

8. Poche drainante (10) de la revendication 7, dans laquelle les premier et second éléments de fermeture (22, 26, 126) sont configurés pour fournir un retour tactile lorsque le premier élément de fermeture (22) s'emboîte correctement sur le second élément de fermeture (26, 126) pour faciliter la fermeture de la décharge sans fuite.

9. Poche de stomie drainante (10) de l'une quelconque des revendications 7 à 8, dans laquelle les première et seconde lignes de pliage (24, 28, 128) sont formées à travers les éléments de fermeture correspondants (22, 26, 126) dans une direction perpendiculaire à la direction de la décharge s'étendant de la cavité.

10. Poche de stomie drainante (10) de l'une quelconque des revendications 4 à 9, dans laquelle les premier et second éléments de fermeture (22, 26, 126) sont configurés pour se plier à l'opposé de la décharge dans des directions opposées.

11. Poche de stomie drainante (10) de l'une quelconque des revendications 1 à 10, dans laquelle la ligne de pliage (24, 28, 128) est formée par une ligne de pli s'étendant à travers l'élément de fermeture correspondant (22, 26, 126) et faisant saillie à l'opposé de la décharge (18, 118), et/ou dans laquelle la ligne de pliage (24, 28, 128) est définie par un pli s'étendant à travers l'élément de fermeture correspondant.

12. Poche de stomie drainante (10) de l'une quelconque des revendications 1 à 11, dans laquelle chacun des au moins un élément de fermeture (22, 26, 126) comporte une ligne de pliage croisée (125, 129) disposée approximativement au milieu de l'élément de fermeture (22, 26, 126) et configurée pour faciliter le pliage de l'élément de fermeture au milieu dans une direction opposée à la décharge lorsqu'une force vers l'intérieur est appliquée aux bords extérieurs de l'élément de fermeture pour ouvrir la décharge (18, 118).

13. Poche de stomie drainante (10) de la revendication 12, dans laquelle la ligne de pliage croisée (125, 129) s'étend verticalement dans la direction de la décharge s'étendant de la cavité (16), et/ou dans laquelle la ligne de pliage croisée est formée par une ligne de pli, une ligne de marquage, une ligne de pliure ou une ligne en relief.

14. Poche de stomie drainante (10) de l'une quelconque des revendications 1 à 13 dans laquelle l'au moins un élément de fermeture (22, 26, 126) est formé à partir d'un matériau polymère.

15. Poche de stomie drainante (10) de l'une quelconque des revendications 1 à 14, comprenant en outre : une languette (138) fixée à une surface de la décharge (18, 118) et configurée pour faciliter l'ouverture de la décharge afin de drainer la cavité.
